# EUROPEAN PATENT SPECIFICATION

(11) **EP 1 482 912 B1**
(45) Date of publication and mention of the grant of the patent: **25.05.2011**
(21) Application number: 03716588.3
(22) Date of filing: 14.03.2003
(51) Int. Cl.: A61K 9/20, A61K 9/24

(54) **NALTREXONE HYDROCHLORIDE COMPOSITIONS**
NALTREXONHYDROCHLORID-ZUSAMMENSETZUNGEN
COMPOSITIONS A BASE DE CHLORHYDRATE DE NALTREXONE

(30) Priority: 14.03.2002 US 364521 P
(43) Date of publication of application: 08.12.2004
(60) Divisional application: 10010921.4
(73) Proprietor: EURO-CELTIQUE S.A., 1653 Luxembourg (LU)
(72) Inventor: OSHLACK, Benjamin, New York, NY 10028 (US); HUANG, Hua-Pin, Englewood Cliffs, NJ 07632 (US); GOLIBER, Philip, Brookfield, CT 06804 (US); MANNION, Richard, Carmel, NY 10502 (US)
(74) Representative: Maiwald, Walter
(86) International application number: PCT/US2003/007932
(87) International publication number: WO 2003/077867

(56) References cited:
- US-A- 4 816 586
- US-A- 5 994 392
- US-A- 6 071 918
- US-B1- 6 271 239
- FERRARI ANNA ET AL: "Serum time course of naltrexone and 6beta-naltrexol level during long term treatment in drug addicts" DRUG AND ALCOHOL DEPENDENCE, vol. 52, no. 3, 1 November 1998 (1998-11-01), pages 211-220, XP002400472 ISSN: 0376-8716
- INTERNET CITATION: 'EMEA Guideline on Stability Testing: Stability Testing of Existing Active Substances and Related Finished Products', [Online] 01 December 2003, Retrieved from the Internet: <URL:http://www.emea.europa.eu/pdfs/human/q wp/012202en.pdf> [retrieved on 2008-06-26]

## Description

### FIELD OF THE INVENTION

The present invention relates to pharmaceutical compositions comprising naltrexone hydrochloride and a stabilizer, and methods of making and using the same.

### BACKGROUND OF THE INVENTION

Naltrexone is an opioid antagonist. The compound and methods for the synthesis of naltrexone are described in U.S. Patent No. 3,332,950. When coadministered with morphine, heroin or other opioids on a chronic basis in a sufficient amount, naltrexone may reduce the incidence of physical dependence to opioids.

WO 01/58451 discloses an oral dosage form comprising an opioid agonist in releasable form and a sequestered opioid antagonist which is substantially not released when the dosage form is administered intact.

EP 0 880 352 describes a method of stabilization of naloxone which prevents the dimerization of naloxone into bisnaloxone.

The pharmacological and pharmacokinetic properties of naltrexone have been evaluated in multiple animal and clinical studies (see, e.g., Gonzalez J P, et al. Naltrexone: A review of its Pharmacodynamic and Pharmacokinetic Properties and Therapeutic Efficacy in the Management of Opioid Dependence. Drugs 1988; 35:192-213). Following oral administration, naltrexone is rapidly absorbed (within 1 hour) and has an oral bioavailability ranging from 5-40%. Naltrexone's protein binding is approximately 21 % and the volume of distribution following single-dose administration is 16.1 L/kg.

Naltrexone hydrochloride is commercially available in tablet form (Revia^{®}, DuPont) for the treatment of alcohol dependence and for the blockade of exogenously administered opioids *(see, e.g.,* Revia, Physician's Desk Reference 51st ed., Montvale, NJ; "Medical Economics" 1997, 51:957-959). A dosage of 50 mg Revia purportedly blocks the pharmacological effects of 25 mg IV administered heroin for up to 24 hours.
WO 01/85257 discloses dosage forms comprising opioid antagonist such as naltrexone in an amount of at least about 0.0001 mg to about 1.0 mg, optionally further comprising a stabilizer

One of the requirements for an acceptable pharmaceutical composition is that it must be stable, so as not to exhibit substantial decomposition of the active ingredient during the time between manufacture of the composition and use by the patient. A number of drugs, for example, are known to undergo hydrolytic decomposition, which is one of the most common routes of drug decomposition. Hydrolytic decomposition can be influenced, e.g., by light, oxidation, and pH.

It has been found that naltrexone hydrochloride may degrade upon storage, possibly due to heat, light, and/or oxygen. Such degradation may have a more pronounced impact on the efficacy of naltrexone hydrochloride when the naltrexone hydrochloride is dosed in smaller amounts than when it is dosed in larger amounts.

There exists a need in the art for naltrexone hydrochloride compositions in an amount of less than 20 mg which inhibit the degradation of, and maintain the stability of the naltrexone hydrochloride.

All documents cited herein, including the foregoing, are incorporated by reference in their entireties for all purposes.

### OBJECTS AND SUMMARY OF THE INVENTION

It is an object of the present invention to provide a composition and method for the stabilization of naltrexone hydrochloride.

It is an object of certain embodiments of the present invention to provide a pharmaceutical composition comprising naltrexone hydrochloride and a pharmaceutically acceptable stabilizer.

It is an object of certain embodiments of the present invention to provide a pharmaceutical composition comprising naltrexone hydrochloride wherein the composition has sufficient stability during the manufacture, storage and dispensing of the naltrexone hydrochloride.

It is an object of certain embodiments of the present invention to provide a composition comprising naltrexone hydrochloride and a method of inhibiting the formation of a degradation product of naltrexone hydrochloride.
The present specification describes a pharmaceutical composition comprising naltrexone hydrochloride in an amount of 20 mg or less, and a stabilizer. In certain embodiments, the amount of naltrexone hydrochloride is greater than .001 mg and less than 20 mg or the amount is greater than .01 mg and less than 20 mg.

In certain embodiments, the present specification describes a pharmaceutical composition comprising naltrexone hydrochloride in an amount of 20 mg or less, and a stabilizer which inhibits the formation of a degradation product from the naltrexone hydrochloride.

In certain embodiments, the present specification describes a pharmaceutical composition comprising naltrexone hydrochloride in an amount of 20 mg or less, and a stabilizer wherein the composition maintains at least 90%, preferably at least 95%, more preferably at least 98%, most preferably at least 99% of the naltrexone hydrochloride in undegraded form after storage of the composition for 1 month at storage conditions of 40±2°C and 75±5% relative humidity.

In certain embodiments, the present specification describes a pharmaceutical composition comprising naltrexone hydrochloride in an amount of 20 mg or less, and a stabilizer wherein the composition maintains at least 90%, preferably at least 95%, more preferably at least 98%, most preferably at least 99% of the naltrexone hydrochloride in undegraded form after storage of the composition for 3 months, preferably for 6 months, at storage conditions of 40±2°C and 75±5% relative humidity.

In certain embodiments, the present specification describes a pharmaceutical composition comprising naltrexone hydrochloride in an amount of 20 mg or less, and a stabilizer wherein the composition maintains at least 90%, preferably at least 95%, more preferably at least 98%, most preferably at least 99% of the naltrexone hydrochloride in undegraded form after storage of the composition for 9 months, preferably for 12 months, and more preferably for 18 months, at storage conditions of 40±2°C and 75±5% relative humidity.

In certain embodiments, the present specification describes a pharmaceutical composition comprising naltrexone hydrochloride in an amount of 20 mg or less, and a stabilizer which inhibits the degradation of the naltrexone hydrochloride, wherein the stabilizer is not BHT.

In certain embodiments, the present specification describes a pharmaceutical composition comprising naltrexone hydrochloride in an amount of 20 mg or less, and a stabilizer which inhibits the degradation of the naltrexone hydrochloride, wherein the naltrexone hydrochloride in combination with the stabilizer is disposed onto a plurality of pharmaceutically acceptable inert beads.

In certain embodiments, the present specification describes a pharmaceutical composition comprising naltrexone hydrochloride in an amount of 20 mg or less, and a water soluble stabilizer which inhibits the degradation of the naltrexone hydrochloride.

In certain embodiments, the present specification describes a pharmaceutical composition comprising naltrexone hydrochloride in an amount of 20 mg or less, a stabilizer, and a chelating agent, wherein at least one of the stabilizer or chelating agent inhibits the degradation of the naltrexone hydrochloride.

In certain embodiments, the present specification describes a pharmaceutical composition comprising naltrexone hydrochloride in an amount of 20 mg or less, and a chelating agent which inhibits the degradation of the naltrexone hydrochloride.

The invention is directed to a pharmaceutical composition comprising an inert core, a first layer and a second layer, the first layer being between the core and the second layer, the first layer comprising naltrexone hydrochloride and a stabilizer and the second layer comprising a hydrophobic material, wherein one or more inert cores (i.e., with the first and second layer) are included in a dosage form to provide a total of 20 mg or less naltrexone hydrochloride, wherein the stabilizer to included in an amount of 0.001 to 2% by weight of the composition.

In certain embodiments, the invention is directed to a pharmaceutical composition comprising an inert core, a first layer, a second layer and a third layer, the first layer being between the core and the second layer, the second layer being between the first layer and the third layer, the first layer comprising naltrexone hydrochloride and a stabilizer, the second layer comprising a first hydrophobic material and the third layer comprising a second hydrophobic material, wherein one or more inert cores (i.e., with the first, second layer and third layer) are included in a dosage form to provide a total of 20 mg or less naltrexone hydrochloride.

In certain embodiments, the present specification describes a pharmaceutical composition comprising about 10 mg oxycodone hydrochloride, less than 5.0 mg naltrexone hydrochloride, and a stabilizer.

In certain embodiments, the present specification describes a pharmaceutical composition comprising a first component comprising about 10 mg oxycodone hydrochloride, and a second component comprising (i) less than 5.0 mg naltrexone hydrochloride and (ii) a stabilizer.

In certain embodiments, the present specification describes a pharmaceutical composition comprising about 20 mg oxycodone hydrochloride, less than 5.0 mg naltrexone hydrochloride, and a stabilizer.

In certain embodiments, the present specification describes a pharmaceutical composition comprising a first component comprising about 20 mg oxycodone hydrochloride, and a second component comprising (i) less than 5.0 mg naltrexone hydrochloride and (ii) a stabilizer.

In certain embodiments, the present specification describes a pharmaceutical composition comprising about 40 mg oxycodone hydrochloride, less than 5.0 mg naltrexone hydrochloride, and a stabilizer.

In certain embodiments, the present specification describes a pharmaceutical composition comprising a first component comprising about 40 mg oxycodone hydrochloride, and a second component comprising (i) less than 5.0 mg naltrexone hydrochloride and (ii) a stabilizer.

In certain embodiments, the present specification describes a pharmaceutical composition comprising 5-20 mg hydrocodone bitartrate, less than 5.0 mg naltrexone hydrochloride, and a stabilizer.

In certain embodiments, the present specification describes a pharmaceutical composition comprising a first component comprising 5-20 mg hydrocodone bitartrate, and a second component comprising (i) less than 5.0 mg naltrexone hydrochloride and (ii) a stabilizer.

The present specification describes also methods of preparing the pharmaceutical compositions as disclosed herein.

The present specification describes also methods of treating a patient comprising administering to the patient a pharmaceutical composition as disclosed herein.

In certain embodiments, the compositions of the present invention comprising naltrexone hydrochloride are capable of being stored over a prolonged period of time at room temperature (e.g., under humidity and temperature conditions usually encountered in pharmacies and in medicine cabinets) without significant degradation.

In certain embodiments of the present invention, the naltrexone hydrochloride and stabilizer of the present invention as defined above are incorporated into a bead composition.

In other embodiments of the invention, part of the naltrexone hydrochloride and stabilizer are in a matrix and/or part of the naltrexone hydrochloride and stabilizer are in one' or more coated beads.

In certain embodiments, the stabilizer is a water-soluble stabilizer, a water-insoluble stabilizer, or mixtures thereof.

In certain embodiments, the naltrexone hydrochloride and the stabilizer of the present invention are sequestered and substantially not released when the dosage form is administered intact, such as disclosed in WO 01/58451.

The compositions of the present invention include, but are not limited to, oral dosage forms such as tablets or capsules. The compositions of the present invention may include any desired pharmaceutical excipients known to those skilled in the art.

The term "naltrexone hydrochloride" is meant to encompass all forms of naltrexone hydrochloride, e.g., the hydrous and anhydrous forms.

The term "disposed about" with respect to an inert bead means that the substance disposed about the bead covers at least a portion of the inert bead, with or without an intermediate layer or layers between the substance and the bead.

### DETAILED DESCRIPTION OF THE INVENTION

An important aspect of all dosage forms is related to the stability of the same. The stability of a pharmaceutical dosage form is related to maintaining its physical, chemical, microbiological, therapeutic, pharmaceutical, and toxicological properties when stored, i.e., in a particular container and environment.

In an additional aspect of certain embodiments of the present invention, the amount of undegraded naltrexone hydrochloride is greater than 90% of its labeled strength, and more preferably greater than 95% percent of the labeled strength after one year of storage under the humidity and temperature conditions usually encountered in pharmacies and medicine cabinets, e.g., room temperature and 35-60% humidity. Thus, when the naltrexone hydrochloride is used in a pharmaceutical preparation, e.g., a tablet, it will retain at least 90% of the naltrexone hydrochloride and preferably at least 95% after one year of storage at room temperature (15°-25° C) at 35-60% humidity.

In certain embodiments, the present invention is directed to a pharmaceutical composition comprising naltrexone hydrochloride and a stabilizer. Preferably the inclusion of the stabilizer inhibits the degradation of the naltrexone hydrochloride by inhibiting the formation of a degradation product. For purposes of the present invention, a degradation product of naltrexone hydrochloride includes for example and without limitation, 10-hydroxynaltrexone; 10-ketonaltrexone; 2,2' bisnaltrexone (pseudonaltrexone); oxides of 2,2' bisnaltrexone; dioxides of 2,2' bisnaltrexone; aldol adduct of naltrexone and 10-hydroxynaltrexone; aldol adduct of naltrexone and 10-ketonaltrexone; naltrexone-N-oxide; 10-hydroxynaltrexone-N-oxide; 10-ketonaltrexone-N-oxide; semiquinones of naltrexone; free radical peroxides of naltrexone; aldol adduct of naltrexone; aldol adducts of naltrexone coupled at the 7,6 position; aldol adducts of naltrexone coupled at the 6,5 position; ether-linked adduct of naltrexone; ether-linked adduct of naltrexone and 10-hydroxynaltrexone; ether-linked adduct of naltrexone and 10-ketonaltrexone; dehydrogenated naltrexone; hydroxy-naltrexone; keto-naltrexone; salts thereof and mixtures thereof; and the like.

Stabilizers of use in this invention include for example, organic acids, carboxylic acids, acid salts of amino acids (e.g., cysteine, L-cysteine, cysteine hydrochloride, glycine hydrochloride or cystine dihydrochloride), sodium metabisulphite, ascorbic acid and its derivatives, malic acid, isoascorbic acid, citric acid, tartaric acid, sodium carbonate, sodium hydrogen carbonate, calcium carbonate, calcium hydrogen phosphate, sulphur dioxide, sodium sulphite, sodium bisulphate, tocopherol, as well as its water- and fat-soluble derivatives, such as e.g., tocofersolan or tocopherol acetate, sulphites, bisulphites and hydrogen sulphites or alkali metal, alkaline earth metal and other metals, PHB esters, gallates, butylated hydroxyanisol (BHA) or butylated hydroxytoluene (BHT), and 2,6-di-t-butyl-alpha-dimethylamino-p-cresol, t-butylhydroquinone, di-t-amylhydroquinone, di-t-butylhydroquinone, butylhydroxytoluene, butylhydroxyanisole, pyrocatechol, pyrogallol, propyl/gallate, and nordihydroguaiaretic acid, as well as lower fatty acids, fruit acids, phosphoric acids, sorbic and benzoic acids as well as their salts, esters, derivatives and isomeric compounds, ascorbyl palmitate, lecithins, mono- and polyhydroxylated benzene derivatives, ethylenediamine-tetraacetic acid and its salts, citraconic acid, conidendrine, diethyl carbonate, methylenedioxyphenols, kephalines, β,β'-dithiopropionic acid, biphenyl and other phenyl derivatives, pharmaceutically acceptable salts thereof, and mixtures thereof. In certain preferred embodiments, the stabilizer is BHT. In other preferred embodiments, the stabilizer is ascorbic acid. All or part of the ascorbic acid can be replaced with a metal or ammonium ascorbate, e.g., sodium, potassium and/or iodine ascorbate(s). Sodium ascorbate is preferred.

In certain embodiments, the stabilizer is selected from the group consisting of organic acids, carboxylic acids, acid salts of amino acids, sodium metabisulphite, ascorbic acid and its derivatives, malic acid, isoascorbic acid, citric acid, tartaric acid, sodium sulphite, sodium bisulphate, tocopherol, water- and fat-soluble derivatives of tocopherol, sulphites, bisulphites and hydrogen sulphites, butylated hydroxyanisol (BHA), 2,6-di-t-butyl-alpha-dimethylamino-p-cresol, t-butylhydroquinone, di-t-amylhydroquinone, di-t-butylhydroquinone, butylhydroxytoluene, butylhydroxyanisole, pyrocatechol, pyrogallol, propyl/gallate, and nordihydroguaiaretic acid, phosphoric acids, sorbic and benzoic acids, esters, derivatives and isomeric compounds, ascorbyl palmitate, pharmaceutically acceptable salts thereof, and mixtures thereof.
The concentration of the stabilizer included in the composition can range from 0.001% to 2% by weight, of the total weight of the naltrexone hydrochloride composition. The present invention extends to the use of combinations of stabilizers especially combinations of the aforementioned stabilizers.

In certain embodiments, the stabilizer is dissolved or dispersed in a solution prior to mixing the stabilizer with the naltrexone hydrochloride. Thereafter, it may be necessary to adjust the pH of the solution or dispersion of the stabilizer to provide for a stabilized naltrexone hydrochloride composition. In certain preferred embodiments, the pH of the solution or dispersion of the stabilizer is adjusted to 3 to 5, preferably about 4.

Many reactions, including many oxidation and decomposition reactions, are catalyzed by trace amounts of metallic ions present in solutions. Many drugs can be degraded through oxidation and hydrolytic reactions which are catalyzed by metal ions. The presence of metallic ions can therefore significantly accelerate the degradation of these drugs. Therefore, chelating agents may also be useful in inhibiting the degradation of naltrexone hydrochloride.

In certain embodiments, chelating agents are included in the compositions of the present invention. In certain embodiments, the chelating agents may be used in addition to or in place of the stabilizers of the present invention. Chelating agents for use in accordance with the present invention, include for example and without limitation, EDTA (methylene diamine tetraacetic acid), a salt of EDTA, desferrioxamine B, deferoxamine, dithiocarb sodium, penicillamine, pentetate calcium, a sodium salt of pentetic acid, succimer, trientine, nitrilotriacetic acid, trans-diaminocyclohexanetetraacetic acid (DCTA), 2-(2-amino-2-oxocthyl)aminoethane sulfonic acid (BES), diethylenetriaminepentaacetic acid, bis(aminoethyl)glycolether-N,N,N,N'-tetraacetic acid, N-2-acetamido-2-iminodiacetic acid (ADA), N-hydroxyethyliminodiacetic acid (HIMDA), N,N-bis-hydroxyethylglycine (bicine), N-(trishydroxymethylmethyl)glycine (tricine), glycylglycine, iminodiacetic acid, citric acid, tartaric acid, fumaric acid, glutamic acid, aspartic acid mixtures thereof, and salts thereof. Preferably the chelating agent is stable, and forms strong metal complexes with a wide variety of metal ions. In addition it is desirable for the chelating agent to be completely non toxic and to have no pharmacological effect on the body except for its chelating effect.

The chelating agent can be present in a concentration of from 0.001 % to 10% by weight; from 0.001% to 5% by weight; or from 0.025% to 2% by weight. Most preferably, the concentration of the chelating agent is from 0.01% to 1% by weight of the total weight of the naltrexone hydrochloride composition.

The present specification also describes a method of manufacturing a pharmaceutical composition comprising: (a) combining naltrexone hydrochloride and a stabilizer in an aqueous solution; (b) optionally adding a chelating agent; and (c) drying the result of step (b) to form a solid or gel pharmaceutical composition. Preferably, the naltrexone hydrochloride and stabilizer are prepared as a particle composition to be incorporated into a dosage form. In certain embodiments, an organic solution can be used instead of or in addition to the aqueous solution.
The particle composition comprising the naltrexone hydrochloride is prepared as coated substrates, such as beads, microspheres, seeds, pellets, ion-exchange resin beads, and other multi-particulate systems. Preferably, substrates coated with the naltrexone hydrochloride and the stabilizer are prepared, e.g., by dissolving the naltrexone hydrochloride and stabilizer in water and then spraying the solution onto a substrate, for example, nu pariel 30/35 beads, using a Wuster insert. Optionally, additional ingredients are also added prior to coating the beads in order to assist the binding of the naltrexone to the beads, and/or to color the solution, etc. For example, a product which includes hydroxypropyl methylcellulose, etc. with or without colorant (e.g., Opadry^{®}, commercially available from Colorcon, Inc.) may be added to the solution and the solution mixed (e.g., for about 1 hour) prior to application of the same onto the substrate. The resultant coated substrate may then be optionally overcoated with a barrier agent as described herein.

Spheroids comprising the naltrexone hydrochloride may also be prepared, for example, by adding a spheronizing agent to the granulation or substrate compositions described above.

In certain embodiments, the naltrexone hydrochloride composition can additionally comprise a diffusion barrier coating. In certain embodiments, the diffusion barrier coating is an enteric coating. The enteric coating includes an anionic polymer such as cellulose acetate phthalate or cellulose acetate trimellatate. An example of a commercially available anionic polymer is Eudragit L30D. Other optional ingredients that can be included in the enteric coating are plasticizers as described herein and antiadherants or glidants such as talc, titanium dioxide, magnesium stearate, silicon dioxide, dibutyl sebacate, ammonium hydroxide, oleic acid colloidal silica, mixtures thereof and the like. In certain embodiments, the diffusion barrier coating prevents the migration of the naltrexone hydrochloride through additional coatings which may be applied to the naltrexone hydrochloride composition.

Pharmaceutical compositions comprising the stabilized naltrexone hydrochloride compositions described herein can be prepared by any conventionally employed means. For example, one or more of above-identified stabilizing agents are added to the naltrexone hydrochloride followed by addition of pharmaceutical auxiliary agents such as excipient, lubricant and disintegrant.

In certain embodiments, wherein the compositions of the present invention further comprises a lubricant, the lubricants for use in the present invention include, for example and without limitation, magnesium stearate, sodium stearate, stearic acid, calcium stearate, magnesium oleate, oleio acid, potassium oleate, caprylic acid, sodium stearyl fumarate, and magnesium palmitate. The optional lubricant to be used in the pharmaceutical products and methods of the invention are substances which are compatible with the stabilizer of the present invention. Generally, the lubricant does not contain groups which could significantly interfere with the function of either the stabilizer component or the drug component.

Generally, the quantity of lubricant present will be from 0.1% to 10%, preferably 0.1% to 5%.

In certain embodiments, the compositions of the present invention further comprise a pharmaceutically acceptable carrier. The carriers which can be used in the instant compositions are also substances which must be compatible with the stabilizer so that they do not interfere with its function in the composition. Generally, the carriers to be used herein are, for example and without limitation, microcrystalline cellulose, polyvinylpyrrolidone, lactose, mannitol, mixtures thereof, and the like. Other examples of pharmaceutically acceptable carriers and excipients that may be used to formulate oral dosage forms are described in the Handbook of Pharmaceutical Excipients, American Pharmaceutical Association (1986), incorporated by reference herein.

In certain embodiments, the compositions of the present invention as defined in claim 1 comprise a controlled release coating. Such controlled release coating may comprise for example an alkylcellulose polymer, an acrylic polymer, or mixtures thereof, as listed below:

### Alkylcellulose Polymers

Cellulosic materials and polymers, including alkylcelluloses, provide hydrophobic materials well suited for coating the beads according to the invention. Simply by way of example, one preferred alkylcellulosic polymer is ethylcellulose, although the artisan will appreciate that other cellulose and/or alkylcellulose polymers may be readily employed, singly or in any combination, as all or part of a hydrophobic coating according to the invention.

One commercially-available aqueous dispersion of ethylcellulose is Aquacoat^{®} (FMC Corp., Philadelphia, Pennsylvania, U.S.A.). Aquacoat^{®} is prepared by dissolving the ethylcellulose in a water-immiscible organic solvent and then emulsifying the same in water in the presence of a surfactant and a stabilizer. After homogenization to generate submicron droplets, the organic solvent is evaporated under vacuum to form a pseudolatex. The plasticizer is not incorporated in the pseudolatex during the manufacturing phase. Thus, prior to using the same as a coating, it is necessary to intimately mix the Aquacoat^{®} with a suitable plasticizer prior to use.

Another aqueous dispersion of ethylcellulose is commercially available as Surelease^{®} (Colorcon, Inc., West Point, Pennsylvania, U.S.A.). This product is prepared by incorporating plasticizer into the dispersion during the manufacturing process. A hot melt of a polymer, plasticizer (dibutyl sebacate), and stabilizer (oleic acid) is prepared as a homogeneous mixture, which is then diluted with an alkaline solution to obtain an aqueous dispersion which can be applied directly onto substrates.

### Acrylic Polymers

In other preferred embodiments of the present invention, the hydrophobic material comprising the controlled release coating is a pharmaceutically acceptable acrylic polymer, including but not limited to acrylic acid and methacrylic acid copolymers, methyl methacrylate copolymers, ethoxyethyl methacrylates, cyanoethyl methacrylate, poly(acrylic acid), poly(methacrylic acid), methacrylic acid alkylamide copolymer, poly(methyl methacrylate), polymethacrylate, poly(methyl methacrylate) copolymer, polyacrylamide, aminoalkyl methacrylate copolymer, poly(methacrylic acid anhydride), and glycidyl methacrylate copolymers.

In certain preferred embodiments, the acrylic polymer is comprised of one or more ammonio methacrylate copolymers. Ammonio methacrylate copolymers are well known in the art, and are described in NF XVII as fully polymerized copolymers of acrylic and methacrylic acid esters with a low content of quaternary ammonium groups.

In order to obtain a desirable dissolution profile, it may be necessary to incorporate two or more ammonio methacrylate copolymers having differing physical properties, such as different molar ratios of the quaternary ammonium groups to the neutral (meth) acrylic esters.

Certain methacrylic acid ester-type polymers are useful for preparing pH-dependent coatings which may be used in accordance with the present invention. For example, there are a family of copolymers synthesized from diethylaminoethyl methacrylate and other neutral methacrylic esters, also known as methacrylic acid copolymer or polymeric methacrylates, commercially available as Eudragit^{®} from Röhm Tech, Inc. There are several different types of Eudragit^{®}. For example, Eudragit^{®} E is an example of a methacrylic acid copolymer which swells and dissolves in acidic media. Eudragit^{®} L is a methacrylic acid copolymer which does not swell at about pH < 5.7 and is soluble at about pH > 6. Eudragit^{®} S does not swell at about pH < 6.5 and is soluble at about pH > 7. Eudragit^{®} RL and Eudragit^{®} RS are water swellable, and the amount of water absorbed by these polymers is pH-dependent, however, dosage forms coated with Eudragit^{®} RL and RS are pH-independent.

In embodiments of the present invention where the coating comprises an aqueous dispersion of a hydrophobic material, the inclusion of an effective amount of a plasticizer in the aqueous dispersion of hydrophobic material will further improve the physical properties of the sustained release coating. For example, because ethylcellulose has a relatively high glass transition temperature and does not form flexible films under normal coating conditions, it is preferable to incorporate a plasticizer into an ethylcellulose coating containing sustained release coating before using the same as a coating material. Generally, the amount of plasticizer included in a coating solution is based on the concentration of the film-former, e.g., most often from about 1 to about 50 percent by weight of the film-former. Concentration of the plasticizer, however, can only be properly determined after careful experimentation with the particular coating solution and method of application.

Examples of suitable plasticizers for ethylcellulose include water insoluble plasticizers such as dibutyl sebacate, diethyl phthalate, triethyl citrate, tributyl citrate, and triacetin, although it is possible that other water-insoluble plasticizers (such as acetylated monoglycerides, phthalate esters, castor oil, etc.) may be used. Triethyl citrate is an especially preferred plasticizer for the aqueous dispersions of ethyl cellulose of the present invention.

Examples of suitable plasticizers for the acrylic polymers of the present invention include, but are not limited to citric acid esters such as triethyl citrate NF XVI, tributyl citrate, dibutyl phthalate, and possibly 1,2-propylene glycol. Other plasticizers which have proved to be suitable for enhancing the elasticity of the films formed from acrylic films such as Eudragit^{®} RL/RS lacquer solutions include polyethylene glycols, propylene glycol, diethyl phthalate, castor oil, and triacetin. Triethyl citrate is an especially preferred plasticizer for the aqueous dispersions of ethyl cellulose of the present invention.

In addition to the above ingredients, the compositions of the present invention may also contain suitable quantities of other materials, e.g., granulating aids, colorants, flavorants and glidants that are conventional in the pharmaceutical art. The quantities of these additional materials will be sufficient to provide the desired effect to the desired composition.

In general, their quantities will be consistent with the amount given above for the drug, and stabilizer, i.e., they make up the remainder of the composition.

The final form of the pharmaceutical preparations made in accordance with the invention can vary greatly. Thus, tablets, caplets, capsules, sachets, and the like are contemplated. Tablets, caplets, and capsules are preferred.

In certain embodiments of the invention, the compositions are film-coated. For example, granules may be film-coated and then either divided into unit doses of naltrexone hydrochloride (e.g., and placed in a gelatin capsule), or compressed into a tablet. Likewise, the tablets prepared in accordance with the invention may be film-coated. Generally, the film-coating substantially comprises a hydrophilic polymer such as hydroxypropylmethylcellulose and does not affect the rate of release of the drug from the composition. The film-coatings which may be used preferably are capable of producing a strong, continuous film that is smooth and elegant, capable of supporting pigments and other coating additives, non-toxic, inert, and tack-free.

The tablet or capsules which incorporate the naltrexone compositions of this invention generally contain 0.01 mg to 20 mg of naltrexone hydrochloride, preferably 0.06 mg to 10 mg, most preferably from 0.1 to 4 mg of naltrexone hydrochloride prepared in accordance with the teachings described herein.

The naltrexone hydrochloride compositions of the present invention can generally be substituted for the naltrexone hydrochloride described in U.S. Patent No.6,277,384; U.S. Patent Nos. 5,512,578; 5,472,943; 5,580,876; or 5,767,125.

### DETAILED DESCRIPTION OF PREFERRED EMBODIMENTS

The following examples and reference examples illustrate various aspects of the present invention.

### EXAMPLE 1 (Reference example)

In Example 1, a naltrexone HCl 0.125 mg composition was prepared having the composition listed in Table 1A:

**TABLE 1A**

| **Ingredients** | **Amt/Unit (mg)** | **Amount/Batch (gm)** |
|---|---|---|
| Naltrexone HCl anhydrous | 0.125 | 0.025 |
| Plasdone C-30 (polyvinylpyrrolidone) | 5.0 | 1.0 |
| Avicel PH-102 (microcrystalline cellulose) | 58.2 | 11.64 |
| Water | 25* | 5.0* |
| Avicel PH-102 | 58.2 | 11.64 |
| Cab-O-Sil (colloidal silicone dioxide) | 0.3 | 0.06 |
| Ac-Di-Sol (croscarmellose sodium) | 2.5 | 0.5 |
| Magnesium Stearate | 0.7 | 0.14 |
| Total | 125 | 25.005 |

| | | |
|---|---|---|
| * removed in the manufacturing process and does not enter into total. | | |

### Process

1. Granulation: Dissolve naltrexone HCl and plasdone C-30 in water. Add the solution to Avicel PH-102 while mixing in collete bowl.
2. Drying: Place the granulation in Glatt GPCG 15 and dry to a mixture level of 5%.
3. Milling: Mill the dried granulation through the CoMil.
4. Mix the granulation with the second quantity of Avicel PH-102, Cab-So-Sil and Ac-Di-Sol.
5. Lubricate the mixture with magnesium stearate.
6. Compression: Compress the lubricated mixture using the Stokes RB2 tablet press.

The composition of Example 1, was tested for stability at temperature of 25±2°C and 60±5% Relative Humidity, over 3 month intervals for up to one year, and gave the following results in Table 1B:

**TABLE 1B**

| **Naltrexone 0.125mg Tablets** | | **25±2°C /60±5% RH 10 count 75cc HDPE Bottles Foil-Lined Heat Induction Sealed Caps** | | | | | |
|---|---|---|---|---|---|---|---|
| Time Point | | Initial | 3 month | 6 month | 9 month | 12 month | 18 month |
| Stability | | | | | | | |
| Test | Limits | | | | | | |
| Assay (naltrexone, % of label) | 90.0-110.0 | 99.9 | 97.5 | 97.6 | 92.2 | 96.5 | 92.8 |
| Total Related Substances | NMT 5.0% | 0.06 | 0.39 | 0.17 | 0.22 | 0.69 | 1.27 |
| Content Uniformity (average %) | 85.0-115.0 | 100.2 | - | - | | | |
| (% RSD of 10 Tablets) | NMT 6.0 | 0.990 | - | - | | | |
| Dissolution (% dissolved) | NLT 90% in 60 minutes | 103 | 103 | 106 | 94 | 95 | 95 |

The composition of Example 1 was tested for stability at temperature of 30±2°C and 60±5% Relative Humidity, at 3 month intervals for up to one year, and gave the following results in Table 1C:

**TABLE 1C**

| **Naltrexone 0.125mg Tablets** | | **30±22°C / 60±5% RH 10 count 75cc HDPE Bottles Foil-Lined Heat Induction Sealed Caps** | | | | |
|---|---|---|---|---|---|---|
| Time Point | | Initial | 3 month | 6 month | 9 month | 12 month |
| Stability | | | | | | |
| Test | Limits | | | | | |
| Assay (naltrexone, % of label) | 90.0-110.0 | 99.9 | 96.9 | 95.5 | 92.4 | 92.7 |
| Total Related Substances | NMT 5.0% | 0.06 | 0.33 | 0.67 | 0.96 | 1.85 |
| Dissolution (% dissolved) | NLT 90% in 60 minutes | 103 | 104 | 102 | 100 | 96 |

The composition of Example 1 was tested for stability at temperature of 40±2°C and 75±5% Relative Humidity, over a 6 month time period and gave the following results in Table 1D:

**TABLE 1D**

| **Naltrexone 0.125mg Tablets** | | **40±2°C / 75±5% RH 10 count 75cc HDPE Bottles Foil-Lined Heat Induction Sealed Caps** | | | | |
|---|---|---|---|---|---|---|
| Time Point | | Initial | 1 month | 2 month | 3 month | 6 month |
| Stability | | | | | | |
| Test | Limits | | | | | |
| Assay (naltrexone, % of label) | 90.0-110.0 | 99.9 | 97.3 | 94.9 | 91.7 | 81.3 |
| Any Individual Unknown | NMT 0.2% | <0.2 | <0.2 | <0.2 | 0.27 | 0.40 |
| Total Related Substances | NMT 1.5% | 0.06 | 0.43 | 1.13 | 1.42 | 1.24 |
| Dissolution (% dissolved) | NLT 90% in 60 minutes | 103 | 95 | 102 | 98 | 87 |

The composition of Example 1 was tested for stability at temperature of 25±2°C and 60±5% Relative Humidity, at 3 month intervals for up to one year, and gave the following results in Table 1E below:

**TABLE 1E**

| **Naltrexone 0.125mg Tablets** | | **25±2°C / 60±5% RH 18 count PVC Blisters** | | | | |
|---|---|---|---|---|---|---|
| **Time Point** | | **Initial** | **3 month** | **6 month** | **9 month** | **12 month** |
| **Stability** | | | | | | |
| Test | Limits | | | | | |
| Assay (naltrexone, % of label) | 90.0-110.0 | 97.9 | 94.6 | 93.0 | 89.1 | 91.9 |
| Total Related Substances | NMT 5.0% | 1.02 | 0.46 | 0.86 | 0.17 | 0.80 |
| Dissolution (% dissolved) | NLT 90% in 60 minutes | 99 | 97 | 88* | 94 | 78 |

| | | | | | | |
|---|---|---|---|---|---|---|
| *Average of S1 and S2 testing must be >85% dissolved in 60 minutes. Passes S2 testing | | | | | | |

The composition of Example 1 was tested for stability at temperature of 40±2°C and 75±5% Relative Humidity, over a 6 month period, and gave the following results in Table 1F below:

**TABLE 1F**

| **Naltrexone 0.125mg Tablets** | | **40±2°C / 75±5% RH 18 count PVC Blisters** | | | |
|---|---|---|---|---|---|
| **Time Point** | | **Initial** | **1 month** | **3 month** | **6 month** |
| **Stability** | | | | | |
| Test | Limits | | | | |
| Assay (naltrexone, % of label) | 90.0-110.0 | 97.9 | 88.6 | 68.2 | 52.6 |
| Total Related Substances | NMT 5.0% | 1.02 | 1.34 | 4.00 | 6.06 |
| Dissolution (% dissolved) | NLT 90% in 60 minutes | 99 | 90 | 64 | 71 |

### EXAMPLE 2 (Reference example)

In Example 2, a naltrexone HCl 0.5 mg composition was prepared having the composition listed below in Table 2A:

**TABLE 2A**

| **Ingredients** | **Amt/Unit (mg)** | **Amount/Batch (gm)** |
|---|---|---|
| Naltrexone HCl anhydrous | 0.5 | 0.1 |
| Plasdone C-30 | 5.0 | 1.0 |
| Avicel PH-102 | 58.0 | 11.6 |
| Water | 25* | 5.0* |
| Avicel PH-102 | 58.0 | 11.6 |
| Cab-O-Sil | 0.3 | 0.06 |
| Ac-Di-Sol | 2.5 | 0.5 |
| Magnesium Stearate | 0.7 | 0.14 |
| Total | 125.0 | 25.0 |

| | | |
|---|---|---|
| * removed in the manufacturing process and does not enter into total. | | |

### Process

The same process as described in Example 1 was used to prepare the naltrexone HCl composition of Example 2.

The composition of Example 2 was tested for stability at temperature of 25±2°C and 60±5% Relative Humidity, at 3 month intervals for up to one year, and gave the following results in Table 2B below:

**TABLE 2B**

| **Naltrexone 0.5mg Tablets** | | **25±2°C / 60±5% RH 10 count 75cc HDPE Bottles Foil-Lined Heat Induction Sealed Caps** | | | | | |
|---|---|---|---|---|---|---|---|
| Time Point | | Initial | 3 month | 6 month | 9 month | 12 month | 18 month |
| Stability | | | | | | | |
| Test | Limits | | | | | | |
| Assay (naltrexone, % of lapel) | 90.0-110.0 | 98.0 | 97.0 | 95.5 | 94.9 | 93.2 | 91.6 |
| Total Related Substances | NMT 5.0% | 0.10 | 0.49 | 0.71 | 0.74 | 0.38 | 2.08 |
| Content Uniformity (average %) | 85.0-115.0 | 96.8 | - | - | - | | |
| (% RSD of 10 Tablets) | NMT 6.0 | 0.97 | - | - | - | - | . |
| Dissolution (% dissolved) | NLT 90% in 60 minutes | 103 | 98 | 95 | 92 | 86 | 95 |

The composition of Example 2 was tested for stability at temperature of 30±2°C and 60±5% Relative Humidity, at 3 month intervals for up to one year, and gave the following results in Table 2C:

**TABLE 2C**

| **Naltrexone 0.5mg Tablets** | | **30±2°C / 60±5% RH 10 count 75cc HDPE Bottles Foil-Lined Heat Induction Sealed Caps** | | | | |
|---|---|---|---|---|---|---|
| Time Point | | Initial | 3 month | 6 month | 9 month | 12 month |
| Stability | | | | | | |
| Test | Limits | | | | | |
| Assay (naltrexone, % of label) | 90.0-10.0 | 98.0 | 96.0 | 94.1 | 92.0 | 90.5 |
| Total Related Substances | NMT 5.0% | 0.10 | 0.85 | 0.93 | 1.44 | 1.02 |
| Dissolution (% dissolved) | NLT 90% in 60 minutes | 103 | 97 | 94 | 92 | 91 |

The composition of Example 2 was tested for stability at temperature of 40±2°C and 75±5% Relative Humidity, over a 6 month period, and gave the following results in Table 2D below:

**TABLE 2D**

| **Naltrexone 0.5mg Tablets** | | **40±2°C / 75±5% RH 10 count 75cc HDPE Bottles Foil-Lined Heat Induction Sealed Caps** | | | | |
|---|---|---|---|---|---|---|
| Time Point | | Initial | 1 month | 2 month | 3 month | 6 month |
| Stability | | | | | | |
| Test | Limits | | | | | |
| Assay (naltrexone, % of label) | 90.0-110.0 | 98.0 | 96.0 | 92.9 | 90.6 | 81.9 |
| Any Individual Unknown | NMT 0.2% | >0.2 | >0.2 | >0.2 | 0.32 | 0.44 |
| Total Related Substances | NMT 5.0% | 0.10 | 0.94 | 1.50 | 2.22 | 2.45 |
| Dissolution (% dissolved) | NLT 90% in 60 minutes | 103 | 96 | 93 | 92 | 83 |

### EXAMPLE 3 (Reference example)

In Example 3, a naltrexone HCl 0.5 mg composition was prepared having the composition listed below in Table 3A:

**TABLE3A**

| **Ingredients** | **Amt/Unit (mg)** |
|---|---|
| Naltrexone HCl anhydrous | 0.5 |
| Plasdone G-30 | 5.0 |
| Avicel PH 102 | 58.0 |
| Water | 25.0 |
| Avicel PH 102 | 58.0 |
| Ac-Di-Sol | 2.5 |
| Cab-O-Sil | 0.3 |
| Magnesium Stearate | 0.7 |
| *Total* | 125.0 |

### Process

The same process as described in Example 1 was used to prepare the naltrexone HCl composition of Example 3.

### EXAMPLE 4 (Reference example)

In Example 4, a naltrexone HCl 0.5 mg composition was prepared as in example 3, substituting stearic acid for magnesium stearate and having the composition listed in Table 4A:

**TABLE 4A**

| **Ingredients** | **Amt/Unit (mg)** |
|---|---|
| Naltrexone HCl anhydrous | 0.5 |
| Plasdone C-30 | 5.0 |
| Avicel PH 102 | 58.0 |
| Water | 25.0 |
| Avicel PH 102 | 57.45 |
| AcDiSol | 2.5 |
| Cab-O-Sil | 0.3 |
| Stearic Acid | 1.25 |
| *Total* | 125.0 |

### EXAMPLE 5 (Reference example)

In Example 5, a naltrexone HCl 0.5 mg composition was prepared as in example 3, with the addition of Sodium Thiosulfate as a stabilizer and having the composition listed below in Table 5A:

**TABLE 5A**

| **Ingredients** | **Amt/Unit (mg)** |
|---|---|
| Naltrexone HCl anhydrous | 0.5 |
| Plasdone C-30 | 5.0 |
| Avicel PH 102 | 57.938 |
| Water | 25.0 |
| Avicel PH 102 | 58.0 |
| Ac-Di-Sol | 2.5 |
| Cab-O-Sil | 0.3 |
| Magnesium Stearate | 0.7 |
| Sodium Thiosulfate | 0.0625 |
| *Total* | 125.0 |

### EXAMPLE 6 (Reference example)

In Example 6, a naltrexone HCl 0.5 mg composition was prepared as in example 3, with the addition of Sodium Metabisulfite as a stabilizer and having the composition listed in Table 6A:

**TABLE 6A**

| **Ingredients** | **Amt/Unit (mg)** |
|---|---|
| Naltrexone HCl anhydrous | 0.5 |
| Plasdone C-30 | 5.0 |
| Avicel PH 102 | 57.938 |
| Water | 25.0 |
| Avicel PH 102 | 58.0 |
| AcDiSol | 2.5 |
| Cab-O-Sil | 0.3 |
| Magnesium Stearate | 0.7 |
| Sodium Metabisulfite | 0.0625 |
| *Total* | 125.0 |

### EXAMPLE 7 (Reference example)

In Example 7, a naltrexone HCl 0.5 mg composition was prepared as in example 3, with the addition of Succinic Acid as a stabilizer and having the composition listed in Table 7A:

**TABLE 7A**

| **Ingredients** | **Amt/Unit (mg)** |
|---|---|
| Naltrexone Hcl anhydrous | 0.5 |
| Plasdone C-30 | 5.0 |
| Avicel PH 102 | 57.875 |
| Water | 25.0 |
| Avicel PH 102 | 58.0 |
| AcDiSol | 2.5 |
| Cab-O-Sil | 0.3 |
| Magnesium Stearate | 0.7 |
| Succinic Acid | 0.125 |
| *Total* | 125.0 |

### EXAMPLE 8 (Reference example)

In Example 8, Examples 3-7 were stored for 2 months under storage conditions of 40° C and 75% relative humidity.

| | **Formula Modification** | **Assay %** | | | **Total Related Substances** | | |
|---|---|---|---|---|---|---|---|
| **Ex. #** | | **Initial** | **1 Month at 40/75** | **2 Month at 40/75** | **Initial** | **1 Month at 40/75** | **2 Month at 40/75** |
| 3 | None (Control) | 100.9 | 91.7 | 88.4 | 0.30 | 0.55 | 1.35 |
| 4 | Stearic Acid | 98.3 | 89.8 | 88.3 | 0.16 | 0.63 | 1.67 |
| 5 | Na+THIOSULFITE | 101.4 | 97.1 | 95.5 | 0.64 | 0.74 | 0.77 |
| 6 | Na+METABISULFITE | 95.3 | 95.3 | 94.8 | 0.17 | 0.34 | 1.05 |
| 7 | SUCCINIC ACID | 97.1 | 90.8 | 88.8 | 0.70 | 0.80 | 1.9 |

| | **Formula Modification** | **10-Keto Naltrexone** | | |
|---|---|---|---|---|
| **Ex. #** | | **Initial** | **1 Month at 40/75** | **2 Month at 40/75** |
| 3 | None (Control) | 0.09 | 0.09 | 0.19 |
| 4 | Stearic Acid | 0.09 | 0.10 | 0.34 |
| 5 | Na+THIOSULFITE | 0.04 | 0.26 | 0.03 |
| 6 | Na+METABISULFITE | ND | 0.04 | 0.01 |
| 7 | SUCCINIC ACID | 0.17 | 0.11 | 0.16 |

Results show that the addition of sodium metabisulfite was significantly more effective than certain other formula modifications in maintaining naltrexone content. Sodium thiosulfite was next best in the ranking followed by succinic acid.

The generation of related substances was also minimized well by sodium metabisulfite but did not perform quite as well as sodium thiosulfite and succinic acid.

Switching lubricants from magnesium stearate to stearic acid did not increase the naltrexone assay value significantly and actually increased related substances nearly twofold.

### EXAMPLE 9 (COMPARATIVE example)

In Example 9, naltrexone HCl controlled release beads were prepared having the composition listed in Table 9A:

**TABLE 9A**

| | Ingredients | Amt/unit (mg) | Amt/batch (g) |
|---|---|---|---|
| Step 1. Drug layering | Naltrexone HCl anhydrous | 0.658 | 12.15 |
| | Non-pareil beads (30/35 mesh) | 79.788 | 1473.0 |
| | Opadry Clear (Hydroxypropymethyl cellulose) | 0.775 | 14.73 |
| Step 2. Anionic polymer coat | Eudragit L30D (dry) | 3.023 | 55.8 |
| | Triethyl Citrate | 0.756 | 13.95 |
| | Glyceryl Monostearate | 0.284 | 5.25 |
| Step 3. Sustained release coat | Eudragit RS30D (dry) | 32.5 | 600.0 |
| | Triethyl citrate | 6.5 | 120.0 |
| | Cab-o-sil | 1.625 | 30.0 |
| Step 4. Seal coat | Opadry Clear (Hydroxypropylmethyl cellulose) | 4.062 | 2325.0 |
| Total (on dry basis) | | 130 | 2400 |

### Bead Manufacturing Process

1. Dissolve naltrexone HCl and Opadry Clear in water. Spray the drug solution onto non-pareil beads in a fluid bed coater with Wurster insert.
2. Disperse Eudragit L30D, Triethyl citrate, and glyceryl monostearate in water. Spray the dispersion onto the drug-loaded beads in the fluid bed coater.
3. Disperse Eudragit RS30D, triethyl citrate, and cabosil in water. Spray the dispersion onto the beads in the fluid bed coater.
4. Dissolve Opadry Clear in water. Spray the solution onto the beads in the fluid bed coater.
5. Cure the beads at 40°C for 24 hours.

The composition of Example 9 was tested for stability at temperature of 40°C and 75 % Relative Humidity, in an open container over a 2 month period, and gave the following results in Table 9B below:

**TABLE 9B**

| | | | |
|---|---|---|---|
| Storage condition 40°C/75%RH (open container) | | | |

| | Initial | 1 month | 2 months |
|---|---|---|---|
| Total related substance (% of Ntx) | 6.8 | 10.3 | 9.4 |
| 10 Keto Naltrexone (% of Ntx) | 3.2 | 7.5 | 6.7 |
| Assay (%) | 101.4 | 87.7 | 85.5 |

### EXAMPLE 10

In Example 10, Naltrexone HCl controlled release beads were prepared as in Example 9, further including BHT as a stabilizer and having the composition listed in Table 10A below:

**TABLE 10A**

| | Ingredients | Amt/unit (mg) | Amt/batch (g) |
|---|---|---|---|
| Step 1. Drug layering | Naltrexone HCl anhydrous | 0.658 | 12.15 |
| | Non-pareil beads (30/35 mesh) | 79.788 | 1473.0 |
| | Opadry Clear (Hydroxypropymethyl cellulose) | 0.775 | 14.31 |
| | BHT | 0.029 | 0.54 |
| Step 2. Anionic polymer | Eudragit L30D (dry) | 3.023 | 55.8 |
| coat | | | |
| | Triethyl Citrate | 0.756 | 13.95 |
| | Glyceryl Monostearate | 0.284 | 5.25 |
| Step 3. Sustained release coat | Eudragit RS30D (dry) | 32.5 | 600.0 |
| | Triethyl citrate | 6.5 | 120.0 |
| | Cabosil | 1.625 | 30.0 |
| Step 4. Seal coat | Opadry Clear (Hydroxypropylmethyl cellulose) | 4.062 | 75.0 |
| Total (on dry basis) | | 130.0 | 2400.0 |

The composition of Example 10 was tested for stability at temperature of 40°C and 75 % Relative Humidity, in an open container over a 1 month period, and gave the following results in Table 10B below:

**TABLE 10B**

| | | | |
|---|---|---|---|
| Storage condition 40°C/75%RH | | | |

| | Initial | 1 month | 2 month |
|---|---|---|---|
| Total related substance (% of Ntx) | 0.12 | 0.85 | 3.91 |
| 10 Keto Naltrexone (% of Ntx) | 0.05 | 0.14 | 0.2 |
| Assay (%) | 103.9 | 95.4 | 99.4 |

### EXAMPLE 11A

In Example 11, Naltrexone HCl controlled release beads were prepared as in Example 9, further including Sodium ascorbate as a stabilizer and EDTA as a chelating agent and having the composition listed in Table 11A below:

**TABLE 11A**

| | Ingredients | Amt/unit (mg) |
|---|---|---|
| Step 1. Drug layering | Naltrexone HCl anhydrous | 0.584 |
| | Non-pareil beads (30/35 mesh) | 80.179 |
| | Opadry Clear (Hydroxypropymethyl cellulose) | 0.341 |
| | Sodium ascorbate | 0.065 |
| | EDTA | 0.065 |
| Step 2. Anionic polymer coat | Eudragit L30D (dry) | 3.023 |
| | Triethyl Citrate | 0.756 |
| | Glyceryl Monostearate | 0.284 |
| Step 3. Sustained release coat | Eudragit RS30D (dry) | 32.5 |
| | Triethyl citrate | 6.5 |
| | Cabosil | 1.625 |
| Step 4. Seal coat | Opadry Clear (Hydroxypropylmethyl cellulose) | 2.438 |
| | Cabosil | 1.625 |
| Total (on dry basis) | | 130 |

The composition of Example 11 was tested for stability at temperature of 40°C and 75 % Relative Humidity, in an open container over a 1 month period, and gave the following results in Table 11B below:

**TABLE 11B**

| | | |
|---|---|---|
| Storage condition 40°C/75%RH | | |

| | Initial | 1 month |
|---|---|---|
| Total related substance (% of Ntx) | 0.1 | 0.1 |
| 10 Keto Naltrexone (% of Ntx) | ND | 0.04 |
| Assay (%) | 112.6 | 108.3 |

### EXAMPLE 12

In Example 12, Naltrexone HCl controlled release beads were prepared as in Example 9, further including ascorbic acid as a stabilizer and having the composition listed in Table 12A below:

**TABLE 12A**

| | Ingredients | Amt/unit (mg) |
|---|---|---|
| Step 1. Drug layering | Naltrexone HCl anhydrous | 0.584 |
| | Non-pareil beads (30/35 mesh) | 80.26 |
| | Opadry Clear (Hydroxypropymethyl cellulose) | 0.341 |
| | Ascorbic acid | 0.065 |
| Step 2. Anionic polymer coat | Eudragit L30D (dry) | 3.023 |
| | Triethyl Citrate | 0.756 |
| | Glyceryl Monostearate | 0.284 |
| Step 3. Sustained release coat | Eudragit RS30D (dry) | 32.5 |
| | Triethyl citrate | 6.5 |
| | Cabosil | 1.625 |
| Step 4. Seal coat | Opadry Clear (Hydroxypropylmethyl cellulose) | 3.532 |
| | Cab-o-sil | 0.531 |
| Total (on dry basis) | | 130.0 |

The composition of Example 12 was tested for stability at temperature of 40°C and 75 % Relative Humidity, in an open container over a 1 month period, and gave the following results in Table 12B below:

**TABLE 12B**

| | | |
|---|---|---|
| Storage condition 40°C/75%RH | | |

| | Initial | 1 month |
|---|---|---|
| Total related substance (% ofNtx) | 0.24 | 0.5 |
| 10 Keto Naltrexone (% of Ntx) | ND | ND |
| Assay (%) | 101.9 | 99.6 |

### EXAMPLE 13

In Example 13, Naltrexone HCl controlled release beads were prepared as in Example 9, further including propyl gallate as a stabilizer and EDTA as a chelating agent and having the composition listed in Table 13A below:

**TABLE 13A**

| | Ingredients | Amt/unit (mg) |
|---|---|---|
| Step 1. Drug layering | Naltrexone HCl anhydrous | 0.61 |
| | Non-pareil beads (30/35 mesh) | 80.211 |
| | Opadry Clear (Hydroxypropymethyl cellulose) | 0.919 |
| | Propyl gallate | 0.00581 |
| | EDTA | 0.00349 |
| Step 2. Anionic polymer coat | Eudragit L30D (dry) | 3.022 |
| | Triethyl Citrate | 0.755 |
| | Glyceryl Monostearate | 0.29 |
| Step 3. Sustained release coat | Eudragit RS30D (dry) | 32.534 |
| | Triethyl citrate | 6.507 |
| | Cab-o-sil | 1.627 |
| Step 4. Seal coat | Opadry Clear (Hydroxypropylmethyl cellulose) | 3.538 |
| | Cab-o-sil | 0.529 |
| Total (on dry basis) | | 130 |

The composition of Example 13 was tested for stability at temperature of 40°C and 75 % Relative Humidity, in an open container over a 1 month period, and gave the following results in Table 13B below:

**TABLE 13B**

| | | |
|---|---|---|
| Storage condition 40°C/75%RH | | |

| | Initial | 1 month |
|---|---|---|
| Total related substance (% of Ntx) | 0.09 | 1.1 |
| 10 Keto Naltrexone (% of Ntx) | ND | 0.18 |
| Assay (%) | 110.5 | 107.4 |

### EXAMPLE 14 (Reference example)

In Example 14, a naltrexone HCl 2.0 mg composition was prepared having the composition listed below in Table 14A:

**TABLE 14A**

| **Ingredients** | **Amt/Unit (mg)** | **Amount/Batch (gm)** |
|---|---|---|
| Naltrexone HCl anhydrous | 2.0 | 116.7 |
| Eudragit RSPO | 88.0 | 5133.3 |
| Stearic Acid | 1,5.0 | 875.0 |
| Stearyl Alcohol | 15.0 | 875.0 |
| Total | 120 | 7000 |

### Process

1. Milling: Mill the stearyl alcohol using a screening mill (Fitzmill).
2. Blending: Blend all ingredients using a convection mixer (V-blender with intensifier bar) at ambient temperature.
3. Extrude the blend with a heating bit set to between 75 and 100 °C into strands approximately 1mm in diameter and cut into cylindrical pellets approximately 1mm in length.

### Stability data

Total naltrexone related substances on completion of manufacture = 2.07%. 10-keto naltrexone = ND.

### EXAMPLE 15 (Reference example)

In Example 15, a naltrexone HCl 2.0 mg composition was prepared having the composition listed below in Table 15A:

**TABLE 15A**

| **Ingredients** | **Amt/Unit (mg)** | **Amount/Batch (gm)** |
|---|---|---|
| Naltrexone HCl anhydrous | 2.0 | 33.0 |
| Eudragit RSPO | 88.0 | 1454.6 |
| Stearic Acid | 15.0 | 248.0 |
| Stearyl Alcohol | 15.0 | 248.0 |
| Citric Acid | 1.0 | 16.6 |
| Total | 121 | 2000 |

### Process

The same process as described in Example 16 was used to prepare the naltrexone HCl composition of Example 15.

### Stability data

Total naltrexone related substances on completion of manufacture = 0.18%. 10-Keto naltrexone =ND.

### EXAMPLE 16 (Reference example)

In Example 16, a naltrexone HCl 2.0 mg composition including BHT as a stabilizer was prepared having the composition listed below in Table 16A:

**TABLE 16A**

| **Ingredients** | **Amt/Unit (mg)** | **Amount/Batch (gm)** |
|---|---|---|
| Naltrexone HCl anhydrous | 2.0 | 100.0 |
| Eudragit RSPO | 88.0 | 4400 |
| Stearic Acid | 15.0 | 750.0 |
| Stearyl Alcohol | 15.0 | 750.0 |
| Butyl hydroxytoluene | 1.0 | 50.0 |
| Total | 121 | 6050 |

### Process

1. Milling: Mill the stearyl alcohol using a screening mill (Fitzmill).
2. Milling: Mill the Butyl hydroxytoluene using a mortar and pestle
3. Blending: Blend all ingredients using a convection mixer (V-blender with intensifier bar) at ambient temperature.
4. Extrude the blend with a heating bit set to between 75 and 100 °C into strands approximately 1mm in diameter and cut into cylindrical pellets approximately 1mm in length.
5. Encapsulation. Encapsulate the pellets into hard gelatin capsules

### Stability data

| | Initial | 1month 40/75 | 3 months 40/75 | 6 months 40/75 |
|---|---|---|---|---|
| Assay | 96.6% | 98.0% | 96.3% | 95.7 |
| Total Related substances | 0.05% | 0.16% | 0.27% | 0.85% |
| 10-Keto Naltrexone | ND | ND | ND | <0.05 |

### EXAMPLE 17 (Reference example)

In Example 17, a naltrexone HCl 2.0 mg composition including BHT as a stabilizer was prepared having the composition listed below in Table 17A:

**TABLE 17A**

| **Ingredients** | **Amt/Unit (mg)** | **Amount/Batch (gm)** |
|---|---|---|
| Naltrexone HCl anhydrous | 2.0 | 100.0 |
| Eudragit RSPO | 96.0 | 4400 |
| Dicalcium Phosphate Anhydrous | 6.0 | 300.0 |
| Stearyl Alcohol | 22.0 | 1100.0 |
| Butyl hydroxytoluene | 1.0 | 50.0 |
| Total | 127 | 6350 |

### Process

The same process as described in Example 16 was used to prepare the naltrexone HCl composition of Example 17.

### Stability data

Compositions prepared in accordance with Example 17 were tested for naltrexone stability under storage conditions of 25 °C/60% relative humidity and 40 °C/75%RH and gave the results listed in Table 17B below:

**TABLE 17B**

| Storage Conditions | Time, Months | Assay, % | Total related substances, % | Water Content, % | 10-Keto Naltrexone |
|---|---|---|---|---|---|
| | | | | | |
| N/A | Initial | 99.4 | <0.05 | 1.18 | ND |
| 25 °C/60%RH | 1 | 99.5 | 0.05 | 1.27 | ND |
| 25 °C/60%RH | 2 | 98.4 | 0.05 | 1.76 | ND |
| 25 °C/60%RH | 3 | 97.9 | 0.06 | 1.55 | ND |
| 25 °C/60%RH | 6 | 98.4 | 0.75 | 1.36 | ND |
| 40 °C/75%RH | 1 | 98.7 | 0.08 | 1.35 | ND |
| 40 °C/75%RH | 3 | 96.3 | 0.14 | 1.83 | ND |
| 40 °C/75%RH | 6 | 96.0 | 1.45 | 1.95 | 0.11 |

### EXAMPLE 18 (Reference example)

In Example 18, a naltrexone HCl 1.0 mg, hydrocodone 10.0mg composition was prepared having the composition listed below in Table 18A:

**TABLE 18A**

| **Ingredients** | **Amt/Unit (mg)** | **Amount/Batch (gm)** |
|---|---|---|
| Naltrexone HCl anhydrous | 1.0 | 10.0 |
| Hydrocodone bitartrate | 10.0 | 100.0 |
| hemipentahydrate | | |
| Povidone K30 | 5.0 | 50.0 |
| Microcrystalline cellulose | 84.0 | 840.0 |
| Water* | N/A | 400* |
| Total | 100 | 1000 |

| | | |
|---|---|---|
| * Not present in final product | | |

### Process

1. Granulation: Dissolve/disperse the naltrexone HCl, hydrocodone bitartrate, and povidone K30 in the water. Add the solution/dispersion to the microcrystalline cellulose while mixing in a high-shear granulator (Colette) at ambient temperature.
2. Drying: Dry the granulation in a fluid-bed-drier (Glatt) with an inlet temperature of 50 to 75°C to approximately equilibrium moisture under ambient conditions.
3. Milling: Mill the granulation using a screening mill (CoMil).
4. Compression: Compress the milled granulation using a single punch tablet press (Manesty F3).

### Stability

Compositions prepared in accordance with Example 18 were tested for naltrexone stability under storage conditions of 50°C for 2 weeks, and 40 °C/75%RH for 1 month and gave the results listed in Table 18B below:

**TABLE 18B**

| Condition | Naltrexone Assay | | 10 Keto-naltrexone | Total Naltrexone Related Substances |
|---|---|---|---|---|
| | Result | % Change* | (%) | % |
| Initial | 112.0 | N/A | ND | 1.79 |
| 2 weeks/50°C | 98.8 | -11.8 | 0.54 | 6.10 |
| 1 Month 40°C/75% RH | 100.0 | - 10.7 | 0.49 | 1.80 |

### EXAMPLE 19 (Reference example)

In Example 19, a naltrexone HCl 1.0 mg, hydrocodone 10.0mg composition, including ascorbic acid as a stabilizer, was prepared having the composition listed below in Table 19A:

**TABLE 19A**

| **Ingredients** | **Amt/Unit (mg)** | **Amount/Batch (gm)** |
|---|---|---|
| Naltrexone HCl anhydrous | 1.0 | 10.0 |
| Hydrocodone bitartrate hemipentahydrate | 10.0 | 100.0 |
| Povidone K30 | 5.0 | 50.0 |
| Ascorbic Acid | 1.0 | 10.0 |
| Microcrystalline cellulose | 83.0 | 830.0 |
| Water* | N/A | 400* |
| Total | 100 | 1000 |

| | | |
|---|---|---|
| * Not present in final product | | |

### Process

1. Granulation: Dissolve/disperse the naltrexone HCl, hydrocodone bitartrate, ascorbic acid and povidone K30 in the water. Add the solution/dispersion to the microcrystalline cellulose while mixing in a high-shear granulator (Colette) at ambient temperature.
2. Drying: Dry the granulation in a fluid-bed-drier (Glatt) with an inlet temperature of 50 to 75°C to a moisture level of 6.1%.
3. Milling: Mill the granulation using a screening mill (CoMil).
4. Compression: Compress the milled granulation using a single punch tablet press (Manesty F3).

### Stability data

Compositions prepared in accordance with of Example 19 were tested for naltrexone stability under storage conditions of 50°C for 2 weeks, and 40 °C/75%RH for 1 month and gave the results listed in Table 19B compared to the results of Example 18:

**TABLE 19B**

| | Example 19 | | Example 18 | | Example 19 | Example 18 |
|---|---|---|---|---|---|---|
| Condition | Naltrexone Assay | | Naltrexone Assay | | Total Naltrexone Related Substances /10-Keto-naltrexone | Total Naltrexone Related Substancess/10-Keto-naltrexone |
| | Result | % Change* | Result | % Change* | (%) | (%) |
| Initial | 108.9 | N/A | 112.0 | N/A | 1.45/ND | 1.79/ND |
| 2 weeks/50C | 106.2 | - 2.5 | 98.8 | -11.8 | 0.57/ND | 6.10/0.54 |
| 1 Month 40C/75%RH | 107.2 | - 1.6 | 100.0 | - 10.7 | 0.78/ND | 1.80/0.49 |

| | | | | | | |
|---|---|---|---|---|---|---|
| * as % of initial | | | | | | |

### EXAMPLE 20 (Reference example)

In Example 20, a naltrexone HCl 0.5 mg, hydrocodone 5.0mg, and acetaminophen 250 mg composition, including ascorbic acid as a stabilizer, was prepared having the composition listed below in Table 20A:

**TABLE 20A**

| Ingredients | Amount/Dosage Unit (mg) | Amount/Batch (g) |
|---|---|---|
| Naltrexone HCl anhydrous | 0.5 | 10.0 |
| Hydrocodone bitartrate hemipentahydrate | 5.0 | 100.0 |
| Povidone K30 | 2.5 | 50.0 |
| Ascorbic acid | 0.5 | 10.0 |
| Microcrystalline cellulose | 41.5 | 830.0 |
| Water* | N/A | 400 * |
| Sub-Total | 50.0 | 1000 |
| | | |
| Milled Granulation | 50.0 | 100.0 |
| DC Acetaminophen (CompapL) ** | 277.8 | 555.5 |
| Magnesium stearate | 2.25 | 4.5 |
| Total | 330 | 660 |

| | | |
|---|---|---|
| * Not present in final product ** CompapL contains 90% acetaminophen | | |

### Process

1. Granulation: Dissolve/disperse the naltrexone HCl, hydrocodone bitartrate, ascorbic acid and povidone K30 in the water. Add the solution/dispersion to the microcrystalline cellulose while mixing in a high-shear granulator (Colette) at ambient temperature.
2. Drying: Dry the granulation in a fluid-bed-drier(Glatt) with an inlet temperature of 50 to 75°C to a moisture level of 6.1%.
3. Milling: Mill the granulation using a screening mill (CoMil).
4. Blending: Blend the milled granulation with the CompapL and magnesium stearate.
5. Compression: Compress the tablets using a single punch tablet press (Manesty F3).

### Stability data

Compositions prepared in accordance with Example 20 were tested for naltrexone stability under storage conditions of 50°C for 2 weeks, and 40 °C/75%RH for 1 month and gave the results listed in Table 20B:

**TABLE 20B**

| | Example 20 | | |
|---|---|---|---|
| Condition | Naltrexone Assay | | Total Naltrexone Related Substances/10-Keto-naltexone |
| | Result | % Change* | % |
| Initial | 107.7 | N/A | 2.32/ND |
| 2 weeks/50C | 106.1 | - 1.5 | 1.61/ND |
| 1 Month 40C/75%RH | 106.4 | - 1.2 | 0.95/ND |

| | | | |
|---|---|---|---|
| * as % of initial | | | |

### EXAMPLE 21 (Reference example)

In Example 21, a naltrexone HCl 1.0 mg, hydrocodone 10.0mg composition, including BHT as a stabilizer, was prepared having the composition listed in Table 21A:

**TABLE 21A**

| Ingredients | Amount/Dosage Unit (mg) | Amount/Batch (g) |
|---|---|---|
| Naltrexone HCl anhydrous | 1.0 | 15.0 |
| Hydrocodone bitartrate hemipentahydrate | 10.0 | 150.0 |
| Povidone K30 | 5.0 | 75.0 |
| Butyl hydroxytoluene | 0.1 | 1.50 |
| Microcrystalline cellulose | 83.9 | 1258.5 |
| Water* | N/A | 400* |
| Total | 100 | 1500 |

| | | |
|---|---|---|
| * not present in final product | | |

### Process

1. Granulation: Dissolve/disperse the naltrexone HCl, butyl hydroxytoluene and povidone K30 in the water. Add the solution/dispersion to the microcrystalline cellulose and hydrocodone bitartrate while mixing in a high-shear granulator (Colette) at ambient temperature.
2. Drying: Dry the granulation in a fluid-bed-drier (Glatt) with an inlet temperature of 50 to 75°C to a moisture level of 2.9%.
3. Milling: Mill the granulation using a screening mill (CoMil).
4. Compression: Compress the milled granulation using a single punch tablet press (Manesty F3).

### Stability data

Compositions prepared in accordance with Example 21 were tested for naltrexone stability under storage conditions of 50 °C for 2 weeks, and 40 °C/75%RH for 1 month and gave the results listed in Table 19B compared to the results of Example 18:

| | Example 21 | | Example 18 | | Example 21 | Example 18 |
|---|---|---|---|---|---|---|
| Condition | Naltrexone Assay | | Naltrexone Assay | | Total Naltrexone Related Substances/ 10-Keto-naltrexone | Total Naltrexone Related Substances/10-Keto-naltrexone |
| | Result | % Change* | Result | % Change * | % | % |
| Initial | 100.2 | N/A | 112.0 | N/A | 0.38/ ND | 1.79/ND |
| 2 weeks/50C | 99.9 | -0.3 | 98.8 | -11.8 | 0.68/ ND | 6.10/0.54 |
| 1 Month 40C/75%RH | 99.9 | - 0.3 | 100.0 | - 10.7 | 1.63/ND | 1.80/0.49 |

| | | | | | | |
|---|---|---|---|---|---|---|
| * as % of initial | | | | | | |

### EXAMPLE 22 (Reference example)

In Example 22, a naltrexone HCl 0.125 mg, hydrocodone 5.0mg, and acetaminophen 500 mg composition, including ascorbic acid as a stabilizer, was prepared having the composition listed in Table 22A:

**TABLE 22A**

| Ingredients | Amount/Dosage Unit (mg) | Amount/Batch (g) |
|---|---|---|
| Granulation | | |
| Naltrexone HCl anhydrous | 0.125 | 1.50 |
| Hydrocodone bitartrate hemipentahydrate | 5.0 | 60.0 |
| Povidone K30 | 4.0 | 48.0 |
| Ascorbic Acid | 1.0 | 12.0 |
| Microcrystalline cellulose | 89.875 | 1078.5 |
| Water* | N/A | 480.0* |
| Sub-Total | 100 | 1200 |
| | | |
| Final Blend | | |
| Milled Granulation | 100 | 228.6 |
| DC Acetaminophen (CompapL)** | 555.6 | 1271.4 |
| Magnesium stearate | 6.6 | 15.0 |
| Total | 662.2 | 1515 |

| | | |
|---|---|---|
| * not present in final product ** CompapL contains 90% acetaminophen | | |

### Process

1. Granulation: Dissolve the naltrexone HCl, ascorbic acid and povidone K30 in the water. Add the solution to the microcrystalline cellulose and hydrocodone bitartrate while mixing in a high-shear granulator (Colette) at ambient temperature.
2. Drying: Dry the granulation in a fluid-bed-drier (Glatt) with an inlet temperature of 50 to 75°C to a moisture level of 4.6%.
3. Milling: Mill the granulation using a screening mill (CoMil).
4. Blending: Blend a portion of the milled granulation with the DC Acetaminophen and the magnesium stearate.
5. Compression: Compress the final blend using a single punch tablet press (Manesty F3).

### Stability data

The composition of Example 22 was tested for naltrexone stability under storage conditions of 50°C for 2 weeks and gave the results listed in Table 22B:

**TABLE 22B**

| | Example 22 | | |
|---|---|---|---|
| Condition | Naltrexone Assay | | Total Naltrexone Related Substances/10-Keto-naltrexone |
| | Result | % Change* | |
| Initial | 100.6 | N/A | 0.95/ND |
| 2 weeks/50C | 97.1 | -3.5 | 6.75/ND |

| | | | |
|---|---|---|---|
| * as % of initial | | | |

### EXAMPLE 23 (Reference example)

In Example 23, a naltrexone HCl 0.125 mg, hydrocodone 5.0mg, and acetaminophen 500 mg composition, including ascorbic acid as a stabilizer, was prepared having the composition listed below in Table 23A:

**TABLE 23A**

| Ingredients | Amount/Dosage Unit (mg) | Amount/Batch (g) |
|---|---|---|
| Granulation | | |
| Naltrexone HCl anhydrous | 0.125 | 1.50 |
| Hydrocodone bitartrate hemipentahydrate | 5.0 | 60.0 |
| Povidone K30 | 5.0 | 60.0 |
| Ascorbic Acid | 1.0 | 12.0 |
| Microcrystalline cellulose | 88.815 | 1065.78 |
| EDTA | 0.060 | 0.72 |
| Water* | N/A | 480.0* |
| Sub-Total | 100 | 1200 |
| Final Blend | | |
| Milled Granulation | 100 | 150 |
| DC Acetaminophen (CompapL)** | 555.6 | 834 |
| Magnesium stearate | 6.6 | 9.89 |
| Total | 662.2 | 984 |

| | | |
|---|---|---|
| * not present in final product ** CompapL contains 90% acetaminophen | | |

### Process

1. Granulation: Dissolve/disperse the naltrexone HCl, ascorbic acid, EDTA and povidone K30 in the water. Add the solution/dispersion to the microcrystalline cellulose and hydrocodone bitartrate while mixing in a high-shear granulator (Colette) at ambient temperature.
2. Drying: Dry the granulation in a fluid-bed-drier (Glatt) with an inlet temperature of 50 to 75°C to a moisture level of 4.6%.
3. Milling: Mill the granulation using a screening mill (CoMil).
4. Blending: Blend a portion of the milled granulation with the DC Acetaminophen and the magnesium stearate.
5. Compression: Compress the final blend using a single punch tablet press (Manesty F3).

### Stability data

The composition of Example 23 was tested for naltrexone stability under storage conditions of 50°C for 2 weeks and gave the results listed in Table 23B:

**TABLE 23B**

| | Example 23 | | |
|---|---|---|---|
| Condition | Naltrexone Assay | | Total Naltrexone Related Substances/10-Keto-naltrexone |
| | Result | % Change* | |
| Initial | 104.2 | N/A | 0.40/ND |
| 2 weeks/50C | 102.6 | -1.5 | 6.46/ND |

| | | | |
|---|---|---|---|
| * as % of initial | | | |

### EXAMPLE 24 (Reference example)

In Example 24, a naltrexone HCl 0.125 mg, hydrocodone 5.0mg, and acetaminophen 500 mg composition, including BHT as a stabilizer, was prepared having the composition listed in Table 24A:

**TABLE 24A**

| Ingredients | Amount/Dosage Unit (mg) | Amount/Batch (g) |
|---|---|---|
| Granulation | | |
| Naltrexone HCl anhydrous | 0.125 | 1.50 |
| Hydrocodone bitartrate hemipentahydrate | 5.0 | 60.0 |
| Povidone K30 | 4.0 | 48.0 |
| Butyl hydroxytoluene | 0.100 | 1.20 |
| Microcrystalline cellulose | 90.775 | 1089.3 |
| Water* | N/A | 480.0* |
| Sub-Total | 100 | 1200 |
| | | |
| Final Blend | | |
| Milled Granulation | 100 | 226.6 |
| DC Acetaminophen (CompapL)** | 555.6 | 1259.8 |
| Magnesium stearate | 6.0 | 13.6 |
| Total | 661.6 | 1500 |

| | | |
|---|---|---|
| * not present in final product ** CompapL contains 90% acetaminophen | | |

### Process

1. Granulation: Dissolve/disperse the naltrexone HCl, butyl hydroxytoluene and povidone K30 in the water. Add the solution/dispersion to the microcrystalline cellulose and hydrocodone bitartrate while mixing in a high-shear granulator (Colette) at ambient temperature.
2. Drying: Dry the granulation in a fluid-bed-drier (Glatt) with an inlet temperature of 50 to 75°C to a moisture level of 4.3%.
3. Screening: Screen the dried granulation through a hand screen.
4. Blending: Blend a portion of the screened granulation with the DC Acetaminophen and the magnesium stearate.
5. Compression: Compress the final blend using a single punch tablet press (Manesty F3).

### Stability data

The composition of Example 24 was tested for naltrexone stability under storage conditions of 50°C for 2 weeks and gave the results listed in Table 24B:

**TABLE 24B**

| | Example 24 | | |
|---|---|---|---|
| Condition | Naltrexone Assay | | Total Naltrexone Related Substances/10-Keto-naltrexone |
| | Result | % Change* | |
| Initial | 91.1 | N/A | 0.71/ND |
| 2 weeks/50C | 92.2 | +1.2 | 0/ND |

| | | | |
|---|---|---|---|
| * as % of initial | | | |

### EXAMPLE 25 (Reference example)

In Example 25, a naltrexone HCl, 0.125 mg, hydrocodone 5.0mg, and acetaminophen 500 mg composition, including BHT as a stabilizer and EDTA, was prepared having the composition listed in Table 25A:

**TABLE 25A**

| Ingredients | Amount/Dosage Unit (mg) | Amount/Batch (g) |
|---|---|---|
| Granulation | | |
| Naltrexone HCl anhydrous | 0.125 | 1.50 |
| Hydrocodone bitartrate hemipentahydrate | 5.0 | 60.0 |
| Povidone K30 | 4.0 | 48.0 |
| Butyl hydroxytoluene | 0.100 | 1.20 |
| EDTA | 0.060 | 0.72 |
| Microcrystalline cellulose | 90.715 | 1088.6 |
| Water* | N/A | 480.0* |
| Sub-Total | 100 | 1200 |
| | | |
| Final Blend | | |
| Milled Granulation | 100 | 226.6 |
| DC Acetaminophen (CompapL)** | 555.6 | 1259.8 |
| Magnesium stearate | 6.0 | 13.6 |
| Total | 661.6 | 1500 |

| | | |
|---|---|---|
| * not present in final product ** CompapL contains 90% acetaminophen | | |

### Process

1. Granulation: Dissolve/disperse the naltrexone HCl, butyl hydroxytoluene, EDTA and povidone K30 in the water. Add the solution/dispersion to the microcrystalline cellulose and hydrocodone bitartrate while mixing in a high-shear granulator (Colette) at ambient temperature.
2. Drying: Dry the granulation in a fluid-bed-drier (Glatt) with an inlet temperature of 50 to 75°C to a moisture level of 3.6%.
3. Screening: Screen the dried granulation through a hand screen.
4. Blending: Blend a portion of the screened granulation with the DC Acetaminophen and the magnesium stearate.
5. Compression: Compress the final blend using a single punch tablet press (Manesty F3).

### Stability data

The composition of Example 25 was tested for naltrexone stability under storage conditions of 50°C for 2 weeks and gave the results listed in Table 25B:

**TABLE 25B**

| | Example 25 | | |
|---|---|---|---|
| Condition | Naltrexone Assay | | Total Naltrexone Related Substances/10-Keto-naltrexone |
| | Result | % Change* | |
| Initial | 91.5 | N/A | 0.66/ND |
| 2 weeks/50C | 93.2 | +1,9 | 0.54/ND |

| | | | |
|---|---|---|---|
| *as % of initial | | | |

### EXAMPLE 26 (Reference example)

In Example 26, a naltrexone HCl 0.125 mg, hydrocodone 5.0mg, and acetaminophen 500 mg composition, including BHT as a stabilizer, was prepared having the composition listed in Table 26A:

**TABLE 26A**

| Ingredients | Amount/Dosage Unit (mg) | Amount/Batch (g) |
|---|---|---|
| Granulation | | |
| Naltrexone HCl anhydrous, | 0.125 | 1.50 |
| Hydrocodone bitartrate hemipentahydrate | 5.0 | 60.0 |
| Acetaminophen | 50.0 | 600.0 |
| Povidone K30 | 5.0 | 60.0 |
| Butyl hydroxytoluene | 0.100 | 1.20 |
| Microcrystalline cellulose | 39.775 | 477.3 |
| Water* | N/A | 480.0* |
| Sub-Total | 100 | 1200 |
| | | |
| Final Blend | | |
| Milled Granulation | 100 | 250.5 |
| DC Acetaminophen (CompapL)** | 500 | 1249.5 |
| Magnesium stearate | 6.0 | 15.0 |
| Total | 606.0 | 1515 |

| | | |
|---|---|---|
| * not present in fmal product ** CompapL contains 90% acetaminophen | | |

### Process

1. Granulation: Dissolve/disperse the naltrexone HCl, butyl hydroxytoluene and povidone K30 in the water. Add the solution/dispersion to the microcrystalline cellulose, acetaminophen and hydrocodone bitartrate while mixing in a high-shear granulator (Colette) at ambient temperature.
2. Drying: Dry the granulation in a fluid-bed-drier (Glatt) with an inlet temperature of 50 to 75°C to a moisture level of 2.9%.
3. Milling: Mill the dried granulation using a screening mill (CoMil).
4. Blending: Blend a portion of the milled granulation with the DC Acetaminophen and the magnesium stearate.
5. Compression: Compress the final blend using a single punch tablet press (Manesty F3).

### Stability data

The composition of Example 26 was tested for naltrexone stability under storage conditions of 50 °C for 2 weeks and gave the results listed in Table 26B:

**TABLE 26B**

| | Example 26 | | |
|---|---|---|---|
| Condition | Naltrexone Assay | | Total Naltrexone Related Substances/10-Keto-naltrexone |
| | Result | % Change* | |
| Initial | 102.7 | N/A | 0.68/ND |
| 2 weeks/50C | 102.4 | -0.3 | 0.41/ ND |

| | | | |
|---|---|---|---|
| * as % of initial | | | |

### EXAMPLE 27 (Reference example)

In Example 27, a naltrexone HCl 0.125 mg, hydrocodone 5.0mg, and acetaminophen 500 mg composition, including BHT as a stabilizer and EDTA, was prepared having the composition listed in Table 27A:

**TABLE 27A**

| Ingredients | Amount/Dosage Unit (mg) | Amount/Batch (g) |
|---|---|---|
| Granulation | | |
| Naltrexone HCl anhydrous | 0.125 | 1.50 |
| Hydrocodone bitartrate hemipentahydrate | 5.0 | 60.0 |
| Acetaminophen | 50.0 | 600.0 |
| Povidone K30 | 5.0 | 60.0 |
| Butyl hydroxytoluene | 0.100 | 1.20 |
| EDTA | 0.060 | 0.72 |
| Microcrystalline cellulose | 39.715 | 476.6 |
| Water* | N/A | 480.0* |
| Sub-Total | 100 | 1200 |
| | | |
| Final Blend | | |
| Milled Granulation | 100 | 250.5 |
| DC Acetaminophen (CompapL)**. | 500 | 1249.5 |
| Magnesium stearate | 6.0 | 15.0 |
| Total | 606.0 | 1515 |

| | | |
|---|---|---|
| * not present in final product ** CompapL contains 90% acetaminophen | | |

### Process

1. Granulation: Dissolve/disperse the naltrexone HCl, butyl hydroxytoluene , EDTA and povidone K30 in the water. Add the solution/dispersion to the microcrystalline cellulose, acetaminophen and hydrocodone bitartrate while mixing in a high-shear granulator (Colette) at ambient temperature.
2. Drying: Dry the granulation in a fluid-bed-drier to a moisture level of 3.5%.
3. Milling: Mill the dried granulation using a screening mill (CoMil).
4. Blending: Blend a portion of the milled granulation with the DC Acetaminophen and the magnesium stearate.
5. Compression: Compress the final blend using a single punch tablet press (Manesty F3).

### Stability data

The composition of Example 30 was tested for naltrexone stability under storage conditions of 50 °C for 2 weeks and gave the results listed in Table 27B:

**TABLE 27B**

| | Example 27 | | |
|---|---|---|---|
| Condition | Naltrexone Assay | | Total Naltrexone Related Substances/10-Keto-naltrexone |
| | Result | % Change* | |
| Initial | 102.5 | N/A | 0.73/ ND |
| 2 weeks/50C | 101.8 | -0.7 | 0.84/ ND |

| | | | |
|---|---|---|---|
| * as % of initial | | | |

## Claims

1. A pharmaceutical composition comprising:
an inert core, a first layer and a second layer, the first layer being between the core and the second layer, the first layer comprising naltrexone hydrochloride and a stabilizer; the second layer comprising a hydrophobic material, wherein one or more inert cores with the first and second layer are included in a dosage form to provide 20 mg or less of naltrexone hydrochloride, wherein the stabilizer is included in an amount of 0.001 to 2% by weight of the composition.

2. The pharmaceutical composition of claim 1, wherein said stabilizer inhibits the formation of a degradation product selected from the group consisting of 10-hydroxynaltrexone; 10-ketonaltrexone; 2,2' bisnaltrexone (pseudonaltrexone); oxides of 2,2' bisnaltrexone; dioxides of 2,2' bisnaltrexone; aldol adduct of naltrexone and 10-hydroxynaltrexone; aldol adduct of naltrexone and 10-ketonaltrexone; naltrexone-N-oxide; 10-hydroxynaltrexone-N-oxide; 10-ketonaltrexone-N-oxide; semiquinones of naltrexone; free radical peroxides of naltrexone; aldol adduct of naltrexone; aldol adducts of naltrexone coupled at the 7,6 position; aldol adducts of naltrexone coupled at the 6,5 position; ether-linked adduct of naltrexone; ether-linked adduct of naltrexone and 10-hydroxynaltrexone; ether-linked adduct of naltrexone and 10-ketonaltrexone; dehydrogenated naltrexone; hydroxy-naltrexone; keto-naltrexone; salts thereof and mixtures thereof.

3. The pharmaceutical composition of claim 1, wherein the pharmaceutically acceptable stabilizer is selected from the group consisting of organic acids, carboxylic acids, acid salts of amino acids, sodium metabisulphite, ascorbic acid and its derivatives, malic acid, isoascorbic acid, citric acid, tartaric acid, sodium carbonate, sodium hydrogen carbonate. calcium carbonate, calcium hydrogen phosphate, sulphur dioxide, sodium sulphite, sodium bisulphate, tocopherol, water and fat-soluble derivatives of tocopherol, sulphites, bisulphites and hydrogen sulphites, or alkali metal, alkaline earth metal and other metals, PHB esters, gallates, butylated hydroxyanisol (BHA) or butylated hydroxytoluene (BHT), 2,6-di-t-butyl-alpha-dimethylamino-p-cresol, t-butylhydroquinone, di-t-amylhydroquinone, di-t-butylhydroquinone, butylhydroxytoluene, butylhydroxyanisole, pyrocatechol, pyrogallol, propyl/gallate, and nordihydroguaiaretic acid, lower fatty acids, fruit acids, phosphoric acids, sorbic and benzoic acids, esters, derivatives and isomeric compounds, ascorbyl palmitate, lecithins, mono- and polyhydroxylated benzene derivatives, citraconic acid, conidendrine, diethyl carbonate, methylenedioxyphenols, kephalines, β,β'-dithiopropionic acid, biphenyl and other phenyl derivatives, pharmaceutically acceptable salts thereof, and mixtures thereof.

4. The pharmaceutical composition of claim 1, further comprising a chelating agent.

5. The pharmaceutical composition of claim 1, wherein the chelating agent is selected from the group consisting of EDTA (ethylene diamine tetraacetic acid), a salt of EDTA, desferrioxamine B, deferoxamine, dithiocarb sodium, penicillamine, pentetate calcium, a sodium salt of pentetic acid, succimer, trientine, nitrilotriacetic acid, trans-diaminocyclohexanetetraacetic acid (DCTA), 2-(2-amino-2-oxocthyl)aminoethane, sulfonic acid (BES), diethylenetriaminepentaacetic acid, bis(aminoethyl)glycolether-N,N,N',N'-tetraacetic acid, N-2-acetamido-2-iminodiacetic acid (ADA), N-hydroxyethyliminodiacetic acid (HIMDA), N,N-bis-hydroxyethylglycine (bicine), N-(trishydroxymethylmethyl)glycine (tricine), glycylglycine, iminodiacetic acid, citric acid, tartaric acid, fumaric acid, glutamic acid, aspartic acid mixtures thereof, and salts thereof.

6. The pharmaceutical composition of claim 1, wherein said composition maintains at least 95% of the naltrexone hydrochloride in undegraded form after storage for 1 month at 40°C ±2°C and 75% ±5% relative humidity.

7. The pharmaceutical composition of claim 1 further comprising a third layer comprising a hydrophobic material, the first layer being between the core and the second layer, the second layer being between the first layer and the third layer.

8. The pharmaceutical composition of claim 1, wherein said inert core is a pharmaceutically acceptable inert bead.

9. The pharmaceutical composition of claim 1, wherein the second layer is a diffusion barrier coating.

10. The pharmaceutical composition of claim 9, wherein the diffusion barrier coating comprises an anionic polymer.

11. The pharmaceutical composition of claim 1, wherein the hydrophobic material is an acrylic polymer.

## Patentansprüche

1. Arzneimittel, umfassend:
einen inerten Kern, eine erste Schicht und eine zweite Schicht, wobei sich die erste Schicht zwischen dem Kern und der zweiten Schicht befindet, die erste Schicht Naltrexonhydrochlorid und einen Stabilisator umfasst, die zweite Schicht ein hydrophobes Material umfasst, wobei einer oder mehrere inerte Kerne mit der ersten und zweiten Schicht in einer Dosierungsform enthalten sind, um 20 mg oder weniger an Naltrexonhydrochlorid bereitzustellen, wobei der Stabilisator in einer Menge von 0,001 bis 2 Gew.-% bezogen auf die Zusammensetzung enthalten ist.

2. Arzneimittel gemäß Anspruch 1, wobei der Stabilisator die Bildung eines Zersetzungsproduktes inhibiert, ausgewählt aus der Gruppe, bestehend aus 10-Hydroxynaltrexon; 10-Ketonaltrexon; 2,2`-Bisnaltrexon (Pseudonaltrexon); Oxide von 2,2`-Bisnaltrexon; Dioxide von 2,2'-Bisnaltrexon; Aldoladdukt von Naltrexon und 10-Hydroxynaltrexon; Aldoladdukt von Naltrexon und 10-Ketonaltrexon; Naltrexon-N-oxid; 10-Hydroxynaltrexon-N-oxid; 10-Ketonaltrexon-N-oxid; Semichinon von Naltrexon; freie Radikalperoxide von Naltrexon; Aldoladdukt von Naltrexon; Aldoladdukte von Naltrexon, die an der 7,6-Position gekuppelt sind; Aldoladducte von Naltrexon, die an der 6,5-Position gekuppelt sind; über Ether gebundene Addukte von Naltrexon; über Ether gebundende Addukte von Naltrexon und 10-Hydroxynaltrexon; über Ether gebundene Addukte von Naltrexon und 10-Ketonaltrexon; dehydriertes Naltrexon; Hydroxy-Naltrexon; KetoNaltrexon; Salze davon und Gemische davon.

3. Arzneimittel gemäß Anspruch 1, wobei der pharmazeutisch verträgliche Stabilisator ausgewählt ist aus der Gruppe, bestehend aus organischen Säuren, Carbonsäuren, sauren Salzen von Aminosäuren, Natriumetabisulfit, Ascorbinsäure und dessen Derivaten, Maleinsäure, Isoascorbinsäure, Zitronensäure, Weinsäure, Natriumcarbonat, Natriumhydrogencarbonat, Kalziumcarbonat, Kalziumhydrogenphosphat, Schwefeldioxid, Natriumsulfid, Natriumbisulphat, Tocopherol, Wasser- und fettlöslichen Derivaten von Tocopherol, Sulfite, Bisulfite und Hydrogensulfite oder Alkalimetalle, Erdalkalimetalle oder andere Metalle, PHB-Ester, Galate, butyliertes Hydroxyanisol (BHA) oder butyliertes Hydroxytoluol (BHT), 2,6-Di-t-butyl-alpha-dimethylamino-p-cresol, t-Butylhydroxykenon, Di-p-amylhydrochinon, Di-t-butylhydroxychinon, Butylhydroxytoluol, Butylhydroxyanisol, Pyrokatechol, Pyrogallol, Propyl/Gallat und Nordihydroguaiarinsäure, niedrige Fettsäuren, Fruchtsäuren, Phosphorsäuren, Sorbin- und Benzoesäuren, Estern, Derivaten und isomeren Verbindungen, Ascorbylpalmitat, Lecithinen, mono- und polyhydroxilierte Benzolderivate, Citraconinsäure, Conidendrin, Diethylcarbonat, Methylendioxidphenole, Kephaline, β,β'-Dithiopropionsäure, Biphenyl- und anderen Phenylderivaten, pharmazeutisch verträglichen Salzen davon und Gemischen davon.

4. Arzneimittel gemäß Anspruch 1, weiterhin ein chelatisierendes Mittel umfassend.

5. Arzneimittel gemäß Anspruch 1, wobei das chelatisierende Mittel ausgewählt ist aus der Gruppe, bestehend aus EDTA (Ethylendiamintretraessigsäure), einem Salz von EDTA, Desferrioxamin B, Deferoxamin, Dithiocarbnatrium, Penicillamin, Pentetatkalzium, einem Natriumsalz von Pentetinsäure, Succimer, Trientin, Nitrilotriessigsäure, Transdiaminocyclohexantetraessigsäure (DCTA), 2-(2-Amino-2-oxoethyl)aminoethan, Sulfonsäure (BES), Diethylentriaminpentaessigsäure, Bis(aminoethyl)glycolether-N,N,N`, N'-tetraessigsäure, N-2-Acetamido-2-iminodiessigsäure (ADA), N-Hydroxyethylliminodiessigsäure (HIMDA), N,N-bis-Hydroxyethylglycin (Bicin), N-(Trishydroxymethylmethyl)glycin (Tricin), Glycylglycin, Iminodiessigsäure, Zitronensäure, Weinsäure, Fumarsäure, Glutaminsäure, Asparatinsäure, Gemischen davon und Salzen davon.

6. Arzneimittel gemäß Anspruch 1, wobei die Zusammensetzung mindestens 95% des Naltrexonhydrochlorids in unzersetzter Form enthält nach Lagerung für einen Monat bei 40°C ± 2°C und 75% ± 5% relative Feuchte.

7. Arzneimittel gemäß Anspruch 1, weiterhin umfassend eine dritte Schicht, umfassend ein hydrophobes Material, wobei sich die erste Schicht zwischen dem Kern und der zweiten Schicht befindet, die zweite Schicht sich zwischen der ersten Schicht und der dritten Schicht befindet.

8. Arzneimittel gemäß Anspruch 1, wobei der inerte Kern ein pharmazeutisch verträgliches inertes Kügelchen ist.

9. Arzneimittel gemäß Anspruch 1, wobei die zweite Schicht eine diffusionshindernde Beschichtung ist.

10. Arzneimittel gemäß Anspruch 9, wobei die diffusionshindernde Beschichtung ein anionisches Polymer umfasst.

11. Arzneimittel gemäß Anspruch 1, wobei das hydrophobe Polymer ein Acrylpolymer ist.

## Revendications

1. Composition pharmaceutique comprenant :
un noyau inerte, une première couche et une deuxième couche, la première couche étant entre le noyau et la deuxième couche, la première couche comprenant du chlorhydrate de naltrexone et un stabilisant ; la deuxième couche comprenant une matière hydrophobe, un ou plusieurs noyaux inertes avec les première et deuxième couches étant inclus dans une forme galénique pour fournir 20 mg ou moins de chlorhydrate de naltrexone, le stabilisant étant inclus en une quantité de 0,001 à 2 % en poids de la composition.

2. Composition pharmaceutique selon la revendication 1, dans laquelle le stabilisant inhibe la formation d'un produit de dégradation sélectionné dans le groupe constitué par la 10-hydroxynaltrexone ; la 10-cétonaltrexone ; la 2,2'-bisnaltrexone (pseudonaltrexone) ; les oxydes de 2,2'-bisnaltrexone ; les dioxydes de 2,2'-bisnaltrexone ; le produit d'addition aldol de la naltrexone et de la 10-hydroxynaltrexone ; le produit d'addition aldol de la naltrexone et de la 10-cétonaltrexone ; le N-oxyde de naltrexone ; le N-oxyde de 10-hydroxynaltrexone ; le N-oxyde de 10-cétonaltrexone ; les semiquinones de naltrexone ; les peroxydes à radicaux libres de naltrexone ; le produit d'addition aldol de la naltrexone ; les produits d'addition aldol de la naltrexone couplés au niveau de la position 7,6 ; les produits d'addition aldol de la naltrexone couplés au niveau de la position 6,5 ; le produit d'addition à liaison éther de la naltrexone ; le produit d'addition à liaison éther de la naltrexone et de la 10-hydroxynaltrexone ; le produit d'addition à liaison éther de la naltrexone et de la 10-cétonaltrexone ; la naltrexone déshydrogénée ; 'hydroxynaltrexone ; la cétonaltrexone ; les sels de ceux-ci et les mélanges de ceux-ci.

3. Composition pharmaceutique selon la revendication 1, dans laquelle le stabilisant pharmaceutiquement acceptable est sélectionné dans le groupe constitué par les acides organiques, les acides carboxyliques, les sels acides d'aminoacides, le métabisulfite de sodium, l'acide ascorbique et ses dérivés, l'acide malique, l'acide isoascorbique, l'acide citrique, l'acide tartrique, le carbonate de sodium, l'hydrogénocarbonate de sodium, le carbonate de calcium, l'hydrogénophosphate de calcium, le dioxyde de soufre, le sulfite de sodium, le bisulfate de sodium, le tocophérol, l'eau et les dérivés liposolubles de tocophérol, les sulfites, les bisulfites et les hydrogénosulfites ou un métal alcalin, un métal alcalinoterreux et d'autres métaux, les esters de PHB, les gallates, l'hydroxyanisol butylé (BHA) ou l'hydroxytoluène butylé (BHT), le 2,6-di-t-butyl-alpha-diméthylamino-p-crésol, la t-butylhydroquinone, la di-t-amylhydroquinone, la di-t-butylhydroquinone, le butylhydroxytoluène, le butylhydroxyanisole, le pyrocatéchol, le pyrogallol, le propyl/gallate et l'acide nordihydroguaiarétique, les acides gras inférieurs, les acides de fruits, les acides phosphoriques, les acides sorbiques et benzoïques, les esters, les dérivés et les composés isomériques, le palmitate d'ascorbyle, les lécithines, les dérivés de benzène mono et polyhydroxylés, l'acide citraconique, la conidendrine, le carbonate de diéthyle, les méthylène-dioxyphénols, les céphalines, l'acide β,β'-dithiopropionique, le biphényle et les autres dérivés phényle, les sels pharmaceutiquement acceptables de ceux-ci et les mélanges de ceux-ci.

4. Composition pharmaceutique selon la revendication 1 comprenant en outre un chélateur.

5. Composition pharmaceutique selon la revendication 1, dans laquelle le chélateur est sélectionné dans le groupe constitué par l'EDTA (acide éthylène diamine tétra-acétique), un sel de l'EDTA, la desferrioxamine B, la déféroxamine, le diéthyldithiocarbamate de sodium, la pénicillamine, le pentétate de calcium, un sel sodique d'acide pentétique, le succimer, la trientine, l'acide nitrilotriacétique, l'acide transdiaminocyclohexane tétra-acétique (DCTA), le 2-(2-amino-2-oxoéthyl)aminoéthane, l'acide sulfonique (BES), l'acide diéthylènetriamine penta-acétique, l'acide bis(aminoéthyl)glycoléther-N,N,N',N'-tétra-acétique, l'acide N-2-acétamido-2-iminodiacétique (ADA), l'acide N-hydroxyéthyliminodiacétique (HIMDA), la N,N-bis-hydroxyéthylglycine (bicine), la N-(trishydroxyméthylméthyl)glycine (tricine), la glycylglycine, l'acide iminodiacétique, l'acide citrique, l'acide tartrique, l'acide fumarique, l'acide glutamique, l'acide aspartique, les mélanges de ceux-ci et les sels de ceux-ci.

6. Composition pharmaceutique selon la revendication 1, dans laquelle ladite composition maintient au moins 95 % du chlorhydrate de naltrexone sous forme non dégradée après conservation pendant 1 mois à 40°C ± 2°C et 75 % ± 5 % d'humidité relative.

7. Composition pharmaceutique selon la revendication 1 comprenant en outre une troisième couche comprenant une matière hydrophobe, la première couche étant entre le noyau et la deuxième couche, la deuxième couche étant entre la première couche et la troisième couche.

8. Composition pharmaceutique selon la revendication 1, dans laquelle ledit noyau inerte est une bille inerte pharmaceutiquement acceptable.

9. Composition pharmaceutique selon la revendication 1, dans laquelle la deuxième couche est un enrobage faisant office de barrière de diffusion.

10. Composition pharmaceutique selon la revendication 9, dans laquelle l'enrobage faisant office de barrière de diffusion comprend un polymère anionique.

11. Composition pharmaceutique selon la revendication 1, dans laquelle la matière hydrophobe est un polymère acrylique.
